# EUROPÄISCHE PATENTSCHRIFT

(11) **EP 0 477 470 B1**
(45) Veröffentlichungstag und Bekanntmachung des Hinweises auf die Patenterteilung: **02.11.1994**
(21) Anmeldenummer: 91109458.9
(22) Anmeldetag: 10.06.1991
(51) Int. Cl.: A61M 1/00, B65D 41/18

(54) **Saugdrainageflasche**
Vacuum drainage bottle
Flacon de drainage à vide

(30) Priorität: 29.08.1990 CH 2811/90
(43) Veröffentlichungstag der Anmeldung: 01.04.1992
(73) Patentinhaber: Genossenschaft VEBO Solothurnische Eingliederungsstätte für Behinderte, CH-4702 Oensingen (CH)
(72) Erfinder: Müller, Walter, CH-4703 Kestenholz (CH); Nützi, Silvan, CH-4855 Wolfwil (CH); Born, Alessandro, CH-4702 Oensingen (CH)
(74) Vertreter: Fillinger, Peter, Dr.

(56) Entgegenhaltungen:
- EP-A- 0 288 679
- AU-B- 519 377
- DE-A- 2 124 100
- DE-A- 2 642 213
- FR-A- 2 334 580

## Beschreibung

Die vorliegende Erfindung bezieht sich auf eine Saugdrainageflasche gemäss dem Oberbegriff des Patentanspruchs 1.

Eine Saugdrainageflasche dieser Art ist aus der europäischen Patentanmeldung EP-A-0 288 679 bekannt. Die bekannte Saugdrainageflasche weist eine Mündung mit einer zylindrischen Aussenfläche und einer ebenen Stirnfläche auf. Die Mündung bildet gegenüber dem Flaschenhals eine hinterschnittene Schulter. Ein Stopfen aus gummielastischem Material weist einen im Querschnitt ringförmigen Mantel auf, der an einem Ende durch eine ein dem Anschluss an eine Drainageleitung dienendes Schlauchstück aufweisende Wand abgeschlossen ist. Diese Wand ist als ebene Dichtungsfläche gestaltet, die beim Evakuieren der Saugdrainageflasche ausschliesslich mit der ebenen Stirnfläche der Flaschenmündung zusammenwirkt. Am anderen Ende weist der Mantel des Stopfens einen inneren, ringförmigen Vorsprung auf, der die Schulter der Mündung der Drainageflasche hintergreift, um den auf die Flasche aufgesetzten Stopfen unverlierbar festzuhalten. Um das Evakuieren zu erleichtern, ist der Innenquerschnitt des Mantels derart gestaltet, dass die Mantelinnenfläche mindestens an einer Stelle des an die Dichtungsfläche angrenzenden Bereichs nicht dicht gegen die zylindrische Aussenfläche der Flaschenmündung anliegt.

Die bekannte Saugdrainageflasche besteht aus Glas und weist die Nachteile auf, dass sie zerbrechlich ist (Implosion unter Vakuum) und zur Sicherstellung hinreichender Evakuierung und Sterilisation der Flasche einen besonders geformten Stopfen benötigt, der ein Abdichten an der Mantelinnenfläche verhindert und dass die zylindrische Mündung der Flasche wegen der hinterschnittenen Schulter verhältnismässig dick sein muss.

Die gattungsfremde EP-A-283 125 offenbart eine besondere Drainageeinrichtung für die Brusthöhle, die den Abfluss der Körperflüssigkeit durch die Schwerkraft ggf. unter Zuhilfenahme eines schwachen Vakuums vorsieht. Sie ist eine Einwegeinrichtung und nicht für den Wiedergebrauch nach einer Dampfsterilisation vorgesehen. Aus diesen Gründen ist sie nicht in der Lage, die Aufgabe bekannter Wunddrainageflaschen zu erfüllen bzw. nicht geeignet, an deren Stelle verwendet zu werden.

Die ebenfalls gattungsfremde FR-A-2 334 580 zeigt mehrere Verschlüsse für Getränkeflaschen, in welche Getränke unter Normaldruck oder als sog. "Sprudel" unter Kohlensäureüberdruck abgefüllt werden. Diese Flaschen haben im Vergleich mit Saugdrainageflaschen zu enge Flaschenhälse, weshalb sie nicht als solche verwendbar sind.

Die vorliegende Erfindung stellt sich die Aufgabe, eine unzerbrechliche, leichte, hitzebeständige Saugdrainageflasche der eingangs erwähnten Art zu schaffen und den Flaschenhals derart zu gestalten, dass das Evakuieren verbessert wird.

Die erfindungsgemässe Saugdrainageflasche weist die im kennzeichnenden Teil der Patentanspruchs 1 angeführten Merkmale auf.

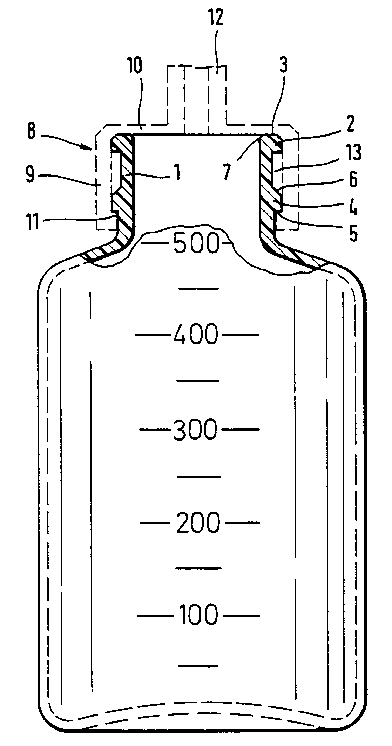

Anhand der beiliegenden schematischen Zeichnung wird die Erfindung beispielsweise erläutert. Die einzige Figur zeigt in Ansicht, teilweise im Schnitt, eine Saugdrainageflasche mit einem angedeuteten aufgesetzten Stopfen.

Die dargestellte Saugdrainageflasche aus druckfestem und hitzebeständigem Kunststoff hat einen Flaschenhals 1, dessen Mündung mit einem äusseren Flansch 2 versehen ist. Der Flansch 2 hat eine ebene Stirnfläche 3. Am Flaschenhals 1 ist ferner eine Ringrippe 4 ausgebildet, welche auf ihrer von der Stirnfläche 3 abgewandten Seite eine Schulter 5 hat. Der Flansch 2 und die Ringrippe 4 haben mindestens angenähert den gleichen Aussendurchmesser, der grösser ist als der Aussendurchmesser des Flaschenhalses 1 zwischen dem Flansch 2 und der Ringrippe 4. Statt der dargestellten geschlossenen Ringrippe 4 können auch mehrere Ringrippenabschnitte ausgebildet sein, die jeweils mit der Schulter 5 versehen sind.

Auf ihrer dem Flansch 2 zugewandten Seite weist die Ringrippe 4 eine Anschrägung 6 auf. Eine gleiche Anschrägung kann auch am Flansch 2 auf seiner der Ringrippe 4 zugewandten Seite ausgebildet sein. Ferner ist die an der Mündung des Flaschenhalses 1 befindliche Innenkante 7 des Flansches 2 abgerundet.

In der Figur ist ein auf den Flaschenhals 1 aufgesetzter Stopfen 8 mit gestrichelten Linien angedeutet. In an sich bekannter Weise hat der aus einem gummielastischen Material bestehende Stopfen 8 einen zylindrischen Mantel 9, der an einem Ende durch eine membranartige Wand 10 abgeschlossen ist und der am andern Ende mit mit einem inneren, die Schulter 5 der Ringrippe 4 hintergreifenden, ringförmigen Vorsprung 11 versehen ist. Die Innenfläche der Wand 10 bildet auf der ebenen Stirnfläche 3 des Flansches 2 des Flaschenhalses 1 eine Dichtungsfläche. An die Wand 10 ist ferner ein Schlauchstück 12 angeformt, das dem Anschluss einer zur Wunde führenden Drainageleitung dient.

Wird die dargestellte Saugdrainageflasche bei aufgesetztem Stopfen 8 mit abgesperrtem Schlauchstück 12 erhitzt, wölbt die sich im Innern der Flasche befindliche Luft die Wand 10 des Stopfens 8 in an sich bekannter Weise nach aussen. Dadurch kann die Luft zwischen der Stirnfläche 3 des Flansches 2 und der Innenfläche der Wand 10 in den ringförmigen Zwischenraum 13 abströmen, der durch den Flansch 2, die Ringrippe 4 und die Innenseite des Mantels 9 begrenzt ist. Da jedoch das mit dem Vorsprung 11 versehene Ende des Mantels 9 an der Ringrippe 4 keine Dichtungsflächen bildet, entweicht die Luft rasch und vollständig nach aussen. Dies ist besonders dann der Fall, wenn in der erwähnten Weise am Flaschenhals 1 statt der geschlossenen Ringrippe 4 mehrere Ringrippenabschnitte ausgebildet sind.

Durch den Sterilisationsprozess und beim Abkühlen der Saugdrainageflasche entsteht im Innern der Flasche ein Vakuum, wodurch die Innenfläche der Wand 10 des Stopfens 8 gegen die Stirnfläche 3 des Flansches 2 des Flaschenhalses 1 gepresst wird, so dass der Stopfen 8 die Drainageflasche vakuumdicht abschliesst. Da sich hierbei die membranartige Wand 10 in das Innere des Flaschenhalses 1 wölbt, schmiegt sich die Innenfläche der Wand 10 an die abgerundete Innenkante 7 des Flansches 3 an, so dass die Dichtungsfläche grösser ist als die eigentliche Stirnfläche 3 des Flansches 1, was die Haltbarkeit des Vakuums verbessert. Das Aufbringen und Abziehen des Stopfens 8 wird durch die Anschrägung 6 der Ringrippe 4 erleichtert.

Die bekannten Saugdrainageflaschen bestehen aus Glas, was den Nachteil einer Bruchgefahr (Implosion), insbesondere während einer Operation, mit sich bringt. Durch die erfindungsgemässe Materialwahl und Ausbildung des Flaschenhalses wird eine bruchsichere (Arbeitssicherheit), hitzebeständige, vakuumfeste und leichte Kunststoff-Saugdrainageflasche möglich.

## Patentansprüche

1. Saugdrainageflasche mit einem Flaschenhals (1), dessen Mündung an der Stirnseite eine ebene Dichtungsfläche (3) für einen topfförmigen, aus gummielastischem Material bestehenden Stopfen (8) bildet, und der eine hinterschnittene Schulter (5) zur Aufnahme eines inneren Vorsprungs (11) des Stopfens (8) aufweist, dadurch gekennzeichnet, dass die Saugdrainageflasche aus einem druckfesten hitzebeständigen Kunststoff besteht, dass die ebene Dichtungsfläche durch eine ebene Stirnfläche (3) eines Flansches (2) des Flaschenhalses (1) und die Schulter (5) durch mindestens eine zur Längsachse des Flaschenhalses (1) konzentrische, äussere Ringrippe (4) gebildet sind, wobei der Flansch (2) und die Ringrippe (4) mindestens angenähert den gleichen Aussendurchmesser haben, der grösser ist als der Aussendurchmesser des Flaschenhalses (1) zwischen dem Flansch (2) und der Ringrippe (4) und dass zwischen dem Flansch (2) und der Ringrippe (4) ein Zwischenraum (13) vorhanden ist, sodaß die bei Erhitzung der Flasche mit aufgesetztem Stopfen (8) an der Stirnfläche des Flansches entweichende Luft sohnell in den Zwischenraum (3) abströmen u. über die Ringrippe (4) nach außen entweichen Kann.

2. Saugdrainageflasche nach Anspruch 1, dadurch gekennzeichnet, dass die einander zugewandten Flächen des Flansches (2) und der Ringrippe (4) schräg zueinander verlaufen.

3. Saugdrainageflasche nach Anspruch 1, dadurch gekennzeichnet, dass die Ringrippe (4) durch Ringrippenabschnitte gebildet ist.

## Claims

1. Suction drainage bottle with a bottle neck (1) whose mouth at the end face forms a plane sealing surface (3) for a cup-shaped stopper (8) made of elastomeric material, and which has an undercut shoulder (5) for accommodating an inner projection (11) of the stopper (8), characterised in that the suction drainage bottle is made of a pressure-resistant heat-stable synthetic plastic material, that the plane sealing surface is formed by a plane end face (3) of a flange (2) of the bottle neck (1) and the shoulder (5) by at least one outer annular rib (4) concentric with the longitudinal axis of the bottle neck (1), the flange (2) and the annular rib (4) having at least approximately the same outer diameter which is greater than the outer diameter of the bottle neck (1) between the flange (2) and the annular rib (4), and that between the flange (2) and the annular rib (4) an intermediate space (13) is provided so that the air escaping at the end face of the flange when the bottle is heated, with the stopper (8) mounted on the bottle, can flow out quickly into the intermediate space (13) and can escape outwardly over the annular rib (4).

2. Suction drainage bottle according to claim 1, characterised in that the mutually facing surfaces of the flange (2) and of the annular rib (4) are disposed at an inclination relatively to one another.

3. Suction drainage bottle according to claim 1, characterised in that the annular rib (4) is formed by annular rib segments.

## Revendications

1. Flacon de drainage par aspiration avec un col de flacon (1), dont le débouché à la face frontale forme une surface plane d'étanchéité (3) pour un bouchon (8) en matériau de caoutchouc élastique en forme de pot, et qui présente un épaulement (5) en contre-dépouille pour recevoir un relief interne (11) du bouchon (8), caractérisé en ce que le flacon de drainage par aspiration est en matière plastique résistant à la pression et à la chaleur, que la surface d'étanchéité plane est constituée d'une face frontale plane (3) d'une bride (2) du col de flacon (1) et que l'épaulement (5) est constitué d'au moins une nervure annulaire (4) extérieure concentrique à l'axe longitudinal du flacon (1), la bride (2) et la nervure annulaire (4) ont au moins de façon approchée le même diamètre extérieur, qui est plus grand que le diamètre extérieur du col de flacon (1) entre la bride (2) et la nervure annulaire (4), et qu'entre la bride (2) et la nervure annulaire (4) existe un espace annulaire (13), de sorte que lors du chauffage du flacon quand le bouchon (8) est mis en place sur la face frontale de la bride, l'air qui s'échappe peut s'écouler rapidement dans l'espace annulaire (13) et s'échapper par la nervure annulaire (4) vers l'extérieur.

2. Flacon de drainage par aspiration selon la revendication 1, caractérisé en ce que les faces (2) de la bride dirigées l'une vers l'autre et de la nervure annulaire (4) sont obliques l'une par rapport à l'autre.

3. Flacon de drainage par aspiration selon la revendication 1, caractérisé en ce que la nervure annulaire (4) est formée de parties de nervure annulaire.
